# EUROPÄISCHE PATENTSCHRIFT

(11) **EP 2 010 906 B1**
(45) Veröffentlichungstag und Bekanntmachung des Hinweises auf die Patenterteilung: **11.06.2014**
(21) Anmeldenummer: 07723802.0
(22) Anmeldetag: 26.03.2007
(51) Int. Cl.: G01N 33/50, G01N 33/53, G01N 33/537

(54) **VERWENDUNG DES 4-1BB REZEPTORS ZUR IDENTIFIZIERUNG UND/ODER SEPARATION AKTIVIERTER REGULATORISCHER TH-ZELLEN (TREG)**
USE OF THE 4-1BB RECEPTOR FOR IDENTIFYING AND/OR SEPARATING ACTIVATED REGULATORY TH CELLS (TREG)
UTILISATION DU RECEPTEUR 4-1BB POUR IDENTIFIER ET/OU SEPARER DES LYMPHOCYTES T AUXILIAIRES REGULATEURS ACTIVÉS (TREG)

(30) Priorität: 24.03.2006 DE 102006014193; 28.03.2006 EP 06090038
(43) Veröffentlichungstag der Anmeldung: 07.01.2009
(73) Patentinhaber: Miltenyi Biotec GmbH, 51429 Bergisch Gladbach (DE)
(72) Erfinder: THIEL, Andreas, 10965 Berlin (DE); SCHÖNBRUNN, Anne, 13055 Berlin (DE); FRENTSCH, Marco, 12161 Berlin (DE)
(74) Vertreter: Boeckh, Tobias
(86) Internationale Anmeldenummer: PCT/EP2007/002860
(87) Internationale Veröffentlichungsnummer: WO 2007/110249

(56) Entgegenhaltungen:
- EP-A- 1 530 972
- WO-A-2004/104185
- WO-A-2005/124346
- US-A1- 2003 049 696
- BD PHARMINGEN TM: "Technical Data Sheet: PE conjugated mouse anti-human CD137 (4-1BB)"[Online] 7. Mai 2005 (2005-05-07), Seiten 1-1, XP002395325 www.bdbiosciences.com Gefunden im Internet: URL:http://www.bdbiosciences.com/external_ files/pm/doc/tds/human/live/web_enabled/36 005X_555956.pdf> [gefunden am 2006-08-18]
- HORI S ET AL: "Control of regulatory T cell development by the transcription factor Foxp3" SCIENCE, AMERICAN ASSOCIATION FOR THE ADVANCEMENT OF SCIENCE,, US, Bd. 299, Nr. 5609, 14. Februar 2003 (2003-02-14), Seiten 1057-1061, XP002316905 ISSN: 0036-8075 in der Anmeldung erwähnt
- ZHENG GUOXING ET AL: "Induction of antitumor immunity via intratumoral tetra-costimulator protein transfer" CANCER RESEARCH, AMERICAN ASSOCIATION FOR CANCER RESEARCH, BALTIMORE, MD, US, Bd. 61, Nr. 22, 15. November 2001 (2001-11-15), Seiten 8127-8134, XP002381948 ISSN: 0008-5472
- PAULY S ET AL: "CD137 IS EXPRESSED BY FOLLICULAR DENDRITIC CELLS AND COSTIMULATES B LUMPHOCYTE ACTIVATION IN GERMINAL CENTERS", JOURNAL OF LEUKOCYTE BIOLOGY, FEDERATION OF AMERICAN SOCIETIES FOR EXPERIMENTAL BIOLOGY, US, vol. 72, no. 1, 1 July 2002 (2002-07-01), pages 35-42, XP009005219, ISSN: 0741-5400
- ANONYMOUS: 'Regulatorische T-Zelle', [Online] 10 Oktober 2009, Seiten 1 - 5 Gefunden im Internet: <URL:http://de.wikipedia.org/wiki/Regulator ische_T-Zelle> [gefunden am 2009-10-22]
- GROUX H: "An overview of regulatory T cells.", MICROBES AND INFECTION / INSTITUT PASTEUR SEP 2001 LNKD- PUBMED:11564435, vol. 3, no. 11, September 2001 (2001-09), pages 883-889, ISSN: 1286-4579

## Beschreibung

Die vorliegend Erfindung betrifft die Verwendung eines 4-1 BB Rezeptors zur Identifizierung und/oder Separation von aktivierten regulatorischen T-Zellen gemäß Anspruch 1 und die Verwendung eines Kits gemäß Anspruch 13, umfassend einen Antikörper zur Detektion des 4-1 BB Rezeptors (CD137) zur Identfikation und/oder Separation aktivierter regulatorischer Th-Zellen, wobei der Antikörper an einen fluoreszierenden Stoff oder magnetische Mikropartikel gekoppelt ist. Die regulatorischen T Zellen können hierbei sowohl polyklonal als auch mit spezifischen Antigenen aktiviert sein. Die Aktivierung kann in vitro oder bereits in vivo erfolgt sein.

Im Organismus existieren spezifische T-Zellen, die nicht erwünschte Immunreaktionen wie zum Beispiel gegenüber körpereigenen Strukturen (Autoantigenen) aktiv unterdrücken. Sie werden als regulatorische T-Zellen (Treg) bezeichnet. Regulatorische T-Zellen sind essentiell and der lebenslangen Erhaltung der peripheren Toleranz gegenüber Autoantigenen beteiligt. Sie werden im Thymus gebildet, dessen Aktivität jedoch mit zunehmendem Alter stark reduziert ist.

Regulatorische T-Zellen können zur Unterdrückung von Anti-Tumor-Antworten führen (Onizuka et al., Cancer Res. 59: 3128-3133 (1999); Shimizu et al., J. Immunol. 163:5211-5218 (1999)), da viele Tumorantigene klassische Autoantigene repräsentieren. Dagegen können verminderte Anzahlen von Treg oder funktionale Veränderungen von Treg zu Autoimmunkrankheiten führen, in deren Verlauf dann körpereigene Strukturen wie Fremdstoffe oder Pathogene unkontrolliert attackiert werden.

Tregs sind an der Aufrechterhaltung der immunologischen Selbsttoleranz beteiligt, indem sie die Aktivierung autoreaktive T-Zellen unterbinden. Sie sind in der Lage, sowohl die Cytokinproduktion als auch die Proliferation solcher potentiell pathogener T-Zellen zu supprimieren. Ein wesentlicher Schritt zur Identifizierung von regulatorischen T-Hetferzellen war die Charakterisierung von CD4+ T-Helferzellen, die die alpha-Kette des Interleukin-2 (IL-2) Rezeptors (CD25) konstitutiv als Oberflächenmembranprotein aufwiesen. Die funktionelle Bedeutung und die genauen molekularen Mechanismen der Suppression dieser CD25+ CD4+ regulatory schen T-Zellen und auch wie sie entstehen, sind bisher nicht geklärt. Da der CD25 Rezeptor auch von Sub-populationen nicht-regutatorischer T-Zeflen ausgeprägt wird, kann dieser Marker nur bedingt zur Analyse und Anreicherung von Treg verwendet werden. Insbesondere aber kann CD25 nicht zur Identifizierung und Separation von aktivierten, Antigen-spezifischen regulatorischen T Zellen verwendet werden.

Eine Isolierung von Treg und im besonderen von Treg spezifisch für bestimmte Antigene ist dennoch eines der grossen therapeutischen Ziele. Ebenso die Analyse von Antigen-spezifischen Treg! (Auto)-Antigen-spezifische Tregs sind ein spezifisches Mittel zur Unterdrückung von ungewolften Immunreaktionen wie z.B. Autoimmunreaktionen bei Rheumatoider-Arthritis (RA), Multipler Sklerose (MS), Atheroskierose (AS), Diabetes, Psoriasis. Andere ungewollte Immunreaktionen bei denen Treg ein spezifsches Mittel darstellen würden sind die GvHD (graft vesus host disease) bei allogene Stamzelltransplantationen oder die Transplantatabstoßung bei Organtransplantationen (Hara et al., J. Immunol. 166: 3789-3796(2001); Taylor et al., J. Exp. Med. 193: 1311-1318 (2001). Auch Allergien stellen ungewollte Immunreaktonen dar, für die es bisher nur wenige therapeutische Optionen gab. Eine therapeutische Behandlung mit Treg, die auf dem natürlichen Prinzip von peripherer Toleranz beruht und in vielen experimentellen Modellen bewiesen ist, würde keine Nebenwirkungen im Vergleich zu klassischen immunosuppressiven Medikamenten beinhalten und wäre kurativ.

Hinsichtlich der aufgeführten Bedeutung der Tregs sind die an der Supression beteiligten Moleküle und Mechanismen sowie zuverlässige Treg-Marker von großer Bedeutung. Im Stand der Technik ist ein molekularbiologischer Treg-Marker, der zur Forkhead-Familie gehörende Transkriptionsrepressor FoxP3, anerkannt (Hori et al (2003) Science 99(5609):1057-61). Des weiteren können regulatorische T-Zellen anhand der Expression des CD25 Moleküls identifiziert werden (Thomton and Shevach (1998) J. Exp. Med. 188 287-296; Sakaguchi et al (1995) J.Immunol. 1551151-1164).

Choi et al 2004, JLB, offenbaren, dass bisher keine Anhaltspunkte existieren, dass 4-1 BB als diskriminativer Marker für Treg gegenüber CD25 negativen Zellen einsetzbar ist.

Allerdings ist es bisher vor allem nicht möglich, die Treg zu identifizieren und zu isolieren, die ein spezifisches Antigen erkennen. Dies wäre möglich, wenn spezifisch aktivierte Treg separiert werden könnten und wäre eine Grundvoraussetzung für spezifische Therapien mit Treg zum Beispiel bei Autoimmunerkrankungen, in denen die der Krankheit zu Grunde liegenden Autoimmunreaktivitäten unterdrückt werden sollten nicht aber Immunreaktivitäten gegen Tumorzellen.

Es ist somit schwierig nach dem bisherigen Stand der Technik regulatorische T-Zellen zu identifizieren und/oder zu isolieren. Bisherige oben beschriebene Marker Lassen nicht zu, dass die Gesamtheit der regulatorischen T-Zellen identifiziert werden kann, da nicht alle regulatorischen T-Zellen definiert spezifische Marker exprimieren. Demzufolge können bisher nur Subpopulationen identifiziert und/oder separiert werden wie zum Beispiel nur die Treg, die den CD25 Rezeptor stark ausprägen. Des Weiteren können verschiedene andere Zellsubpopulationen die gleichen Zelloberflächenmarker besitzen (wie CD4 für Th1 Th2, oder anderen Th-Zellen oder CD25 für aktivierte T-Zellen oder B-Zellen.
Insbesondere ist jedoch die Identifizierung aktivierter Treg nach Stimulation mit definierten Antigenen anhand spezifischer Aktivierungsmarker nicht möglich. Die Aktivierung von regulatorischen Th-Zellen führt zur zwar zur verstärkten Expression von CD25 und CD38, die aber wie alle anderen beschriebenen T-Zellaktivierungsmarker auch in anderen T-Zellen exprimiert werden, so dass bisher keine Antigenspezifischen Treg anhand spezifischer Aktivierungsmarker separiert werden können.

Die Mehrzahl der Methoden, die anhand der Expression eines bestimmten Zelloberflächenmarkers regulatorische Th-Zellen identifizieren und/oder isolieren hängen von der Erkennung eines Markers und der Bindung eines Antikörpers ab. Wenn nicht ein einziger Marker verwendet werden kann, um einen bestimmten Zelltyp zu identifizieren und/oder zu isolieren, muss eine Kombination von Markern und den zu ihnen passenden Antikörpern gefunden werden (Levingset al., J Exp. Med. 193 (11): 1295-1302 (2001)). Solche Experimente können in der Praxis sehr komplex und schwierig in der Durchführung sein. Des Weiteren kann die Bindung der Antikörper die Aktivität der Zielzelle oder die Expression der anderen Marker beeinflussen, so dass der Identifikation und/oder Separationsprozess mit den weiteren Antikörpern negativ beeinflusst wird.

Abschließend soll noch angemerkt werden, dass es durch die Bindung von Antikörpern zu einer Querverbindung (cross-linking), Kappung und Internalisation der betreffenden Marker kommen kann. Hierdurch kann die Zellfunktion eingeschränkt werden. Auch wenn Fab-Fragmente verwendet werden, sind die betreffenden Marker maskiert und können unter Umständen in ihrer Funktion beeinträchtigt sein. Die Bindung an bestimmte Marker kann auch zur Deaktivierung der Zelle führen (CD28, go3), Apoptose induzieren (Quer-vernetzung von CD4 in aktivierten Zellen) oder die Sekretion von Zytokinen induzieren (CD3, CD2, CD28). Anhand des Standes der Technik konnte bisher keine spezifische Identifizierung und/oder Separation von ausschließlich lebenden regulatorischen T-Zellen durchgeführt werden. Der einzig bisher beschriebene spezifische Marker FoxP3 kann zudem neueren Untersuchungen folgend auch in nicht-regulatorischen T-Zellen induziert werden (Fontenot J.D. 2003, Hori S. et al. 2003). Da FoxP3 als intrazelluläres Protein vorliegt, ist es darüberhinaus nach dem bisherigen Stand der Technik nicht möglich, lebende regulatorische Zellen zum Beispiel anhand eines FoxP3-Antikörpers zu identifizieren und/oder zu isolieren. Wiederum stellt sich zusätzlich das Problem, dass nur die Gesamheit der Treg identifiziert werden kann, nicht aber Treg spezifisch für zuvor definierte Antigene. Daher ist eine zuverlässige Identifikation und/oder Separation von Treg und inbesondere Antigen-spezifischer Treg bisher nicht möglich.

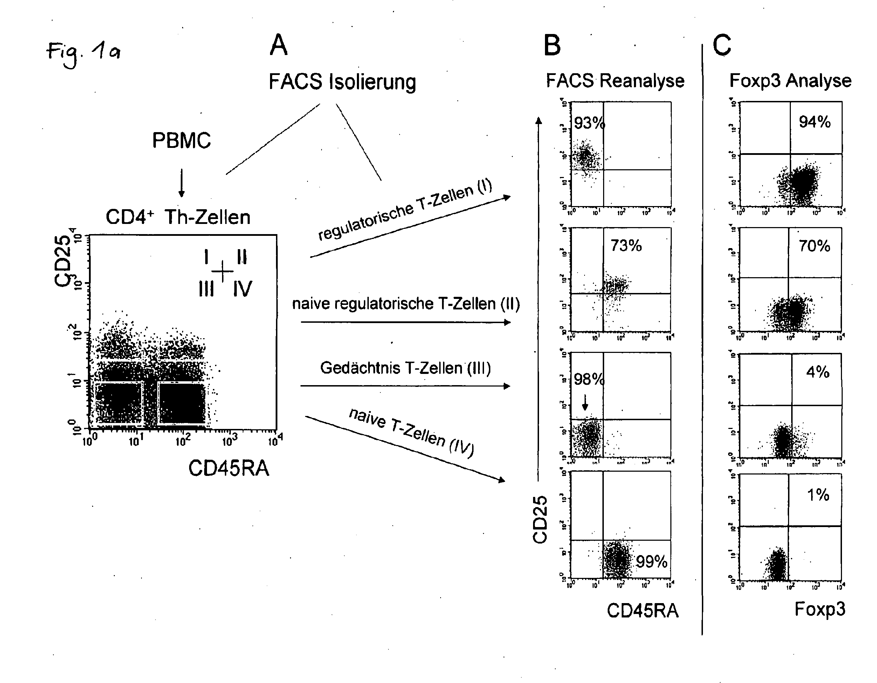

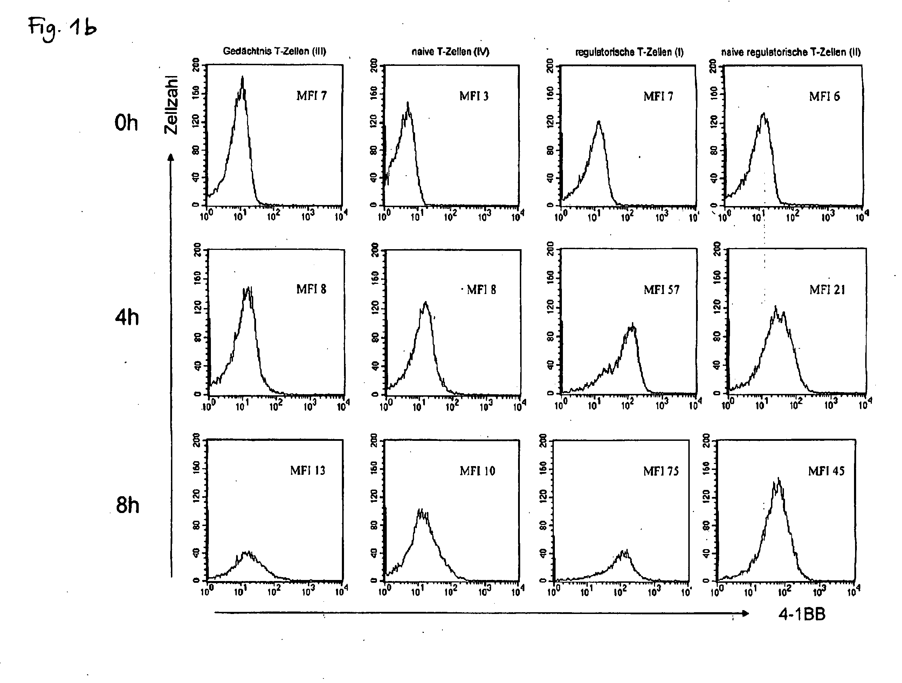

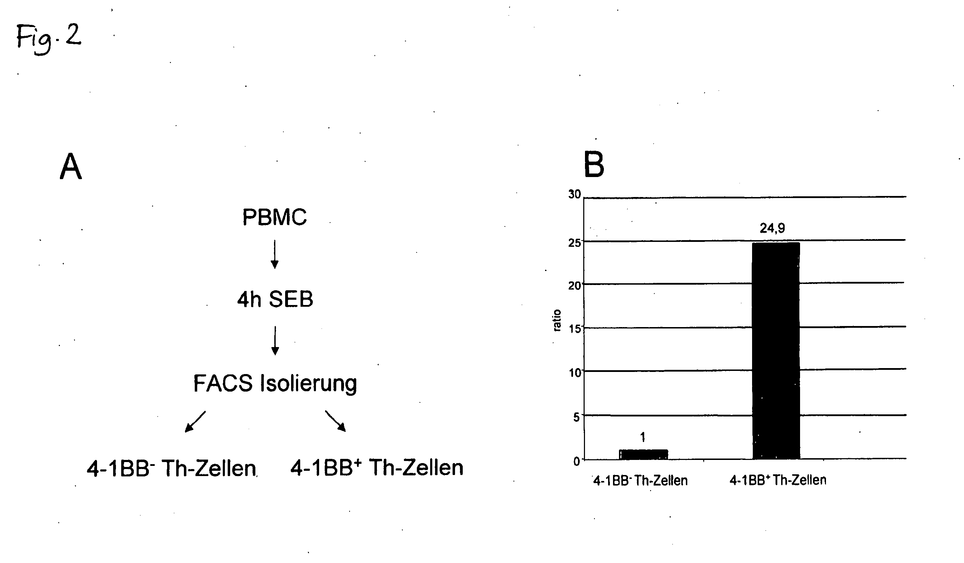

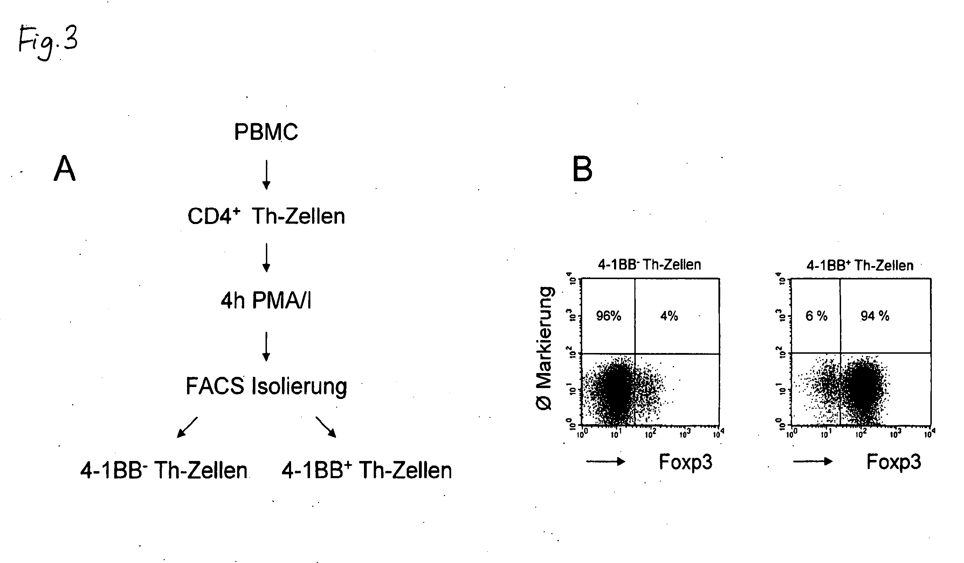

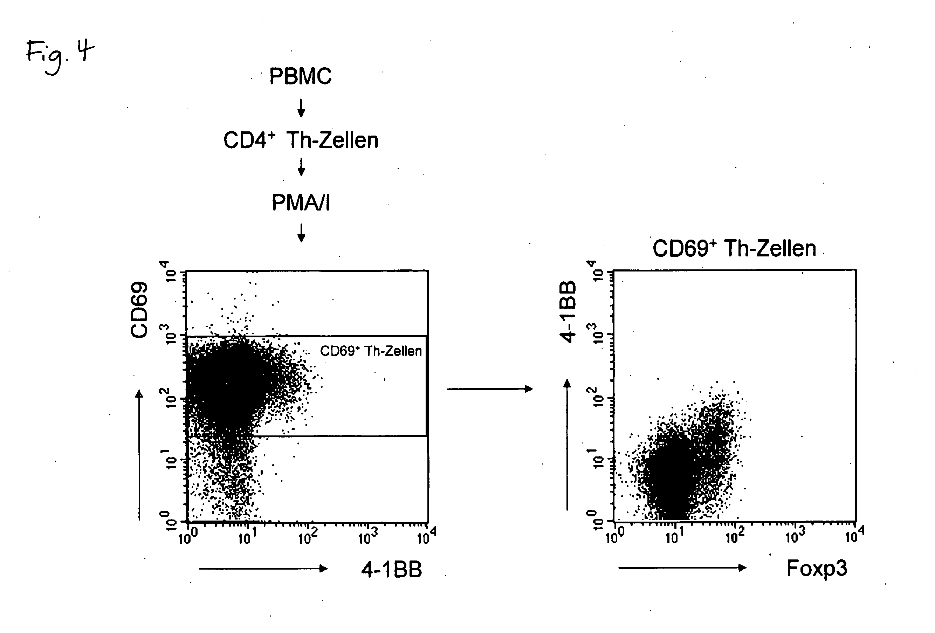

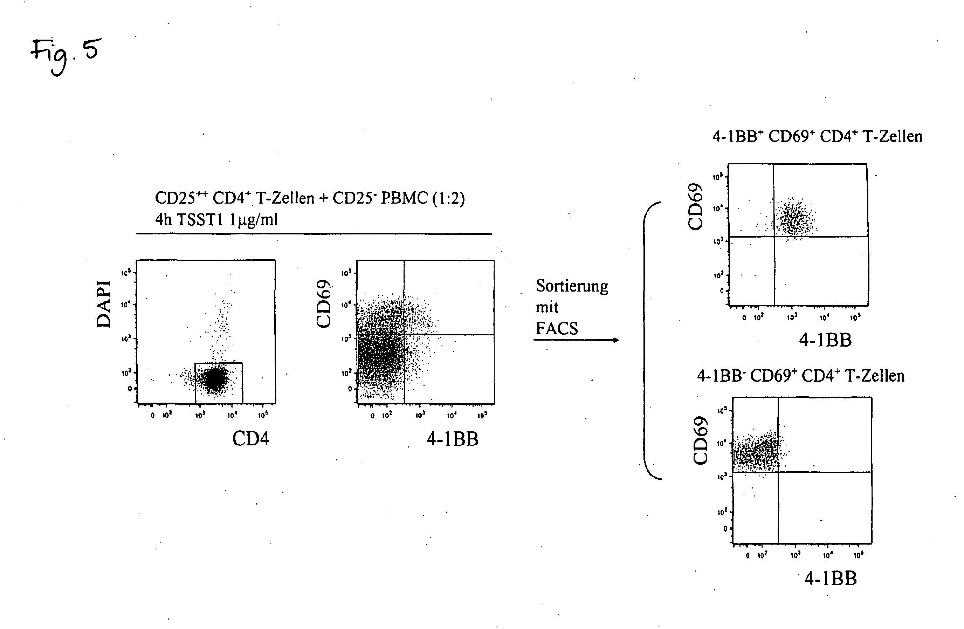

So offenbart auch BD Pharmingen TM: "Technical Data Sheet PE conjugated mouse anti-human CD137 (4-1BB)", 7. Mai 2005, http://Mww.bdbiosciences.com/external_files/pm/doc/tds/human/live/ web_enabled/36005X_555956.pdf, dass der 4-1BB Marker ein Aktivierungsmarker für alle T-Zellen darstellt Auch die WO 2005/124346 beschreibt lediglich, dass 4-1BB Marker auf CD4⁺ CD25⁺ sind; die Druckschrift offenbart allerdings nicht, dass CD4⁺ CD25⁺ zu einem bestimmten Zeitpunkt der Aktivierung 4-1BB Marker im Vergleich zu CD4⁺ CD25⁻ T-Zellen spezifisch ausprägen. Der bestimmte Zeitpunkt kann beispielsweise durch Kontrollen, bevorzugt regelmäßige Kontrollen, ermittelt werden. Es ist beispielsweise möglich, dass im wesentlichen in 30 Minuten Abständen eine Probenentnahme oder eine direkte Beobachtung dahingehend erfolgt, ob die aktivierten regulatorischen Th-Zellen den Marker als diskriminativen Marker ausgeprägt haben. Die mehrfachen, bevorzugt regelmäßigen Kontrollen sind demgemäß regelmäßige Beobachtungen des Systems, direkt durch einen Fachmann oder mithilfe von Analysegeräten oder -mechanismen in Abständen von 5, 10, 15, 30, 45 Minuten, bevorzugt einstündigen Abständen. Es ist also durch die Kontrollen der Zeitpunkt zu bestimmen, an welchem nur die aktivierten regulatorischen Zellen den Rezeptor/Marker aktivieren, wobei es überraschend war, dass dies innerhalb eines kurzen Zeitraums, von ungefähr 3-8 Stunden, vor allem von im wesentlichen bzw. ungefähr 4 Stunden erfolgt Diese Zeitangaben beziehen sich auf die dem Fachmann üblichen Laborbedingungen für die Aktivierung von T-Zellen. Werden deutlich höhere oder niedrigere oder auch nur geringfügig höhere oder niedrigere Temperaturen gewählt, oder aber Zusätze ins System gegeben, die eine Aktivierung hemmen oder stimulieren, kann sich der Zeitraum verlängem oder aber verkürzen. Dadurch, dass beispielsweise ein Fachmann die Ausbildung des Markers/Rezeptors kontrolliert, ist eine einfache Bestimmung der spezifischen zeitlichen Ausprägung möglich. Selbstverständlich weiß der Fachmann, dass er bei Entnahme der T-Zellen aus in vivo Systemen in einem Zellgemisch immer einige Zellen vorfindet, die bereits aktiviert sind und demgemäß 4-1BB (Rezeptor) ausbilden. Die Autoren der WO 2005/124346 offenbaren vielmehr, dass beide Zellpopulationen CD4⁺ CD25⁺ wie auch CD4⁺ CD25⁻ T-Zellen nach ihrer Aktivierung 4-1 BB Marker in gleicher Stärke ausprägen. Die genannten Druckschriften geben damit die Auffassung des Standes der Technik wieder, nach welchem 4-1BB Marker nicht als diskriminativer Marker für CD25⁺ Treg-Zellen im Vergleich zu CD25⁻ T-Zellen verwendet werden kann. Insbesondere beschreibt BD Pharmingen TM einen PE konjugierten Maus anti-human CD137 Antikörper. In der WO 2005/124346 wird vor allem die Co-Stimulation von frisch isolierten CD4⁺ CD25⁺ Maus-Treg-Zellen über den 4-1BB Rezeptor offenbart Die Treg-Zellen werden nicht über den 4-1BB Rezeptor stimuliert, sondern über die Anwesenheit von starken Aktivierungsstimuli wie CD3. Gemäß der genannten Offenbarungen geht ein Fachmann davon aus, dass ein Großteil der so genannten frischen Treg-Zellen 4-1BB nicht ausprägt So ist beispielsweise in Figur 5 der WO 2005/124346 gezeigt, dass ein Co-Stimulation mit 4-1BBL vor allem auch auf CD25- Zellen aktivierende Eigenschaften ausübt Eindeutig wird in Abbildung 1D offenbart, dass CD4⁺ CD25⁺ und CD4⁺ CD25 T-Zellen beide während der Aktivierung den 4-1BB Rezeptor ausprägen, so dass die genannten Druckschriften den Fachmann nicht motivieren, 4-1BB Rezeptoren zur spezifischen Darstellung oder Isolierung von CD4⁺ CD25⁺ Treg-Zellen aus Zellgemischen zu benutzen.

Es wurde erfindungsgemäß überraschend ein Verfahren gefunden, wodurch man lebende, aktivierte regulatorische Th-Zellen (Treg) identifizieren und/oder isolieren kann, wobei man den 4-1BB-Rezeptor als Marker und 4-1BB-spezifische Antikörper zur Identifikation und/oder Isolierung der aktivierten, regulatorische T-Zellen verwendet

Die Erfindung betrifft also die überraschende Lehre, dass insbesondere Antigen-spezifisch aktivierte CD4⁺ CD25⁺ Treg von gleichsam, insbesondere aktivierten CD4⁺ CD25⁻ T-Zellen mittels des 4-1BB Rezeptors isoliert werden können. Demgegenüber wird im Stand der Technik regelmäßig herausgestellt, dass 4-1BB ein Aktivierungsmarker für CD4⁺ CD25⁺ und für CD4⁺ CD25⁺ T-Zellen ist Vor allem weil in keiner der o. g. Schriften des Standes der Technik eine spezifische Ausprägung von 4-1BB auf CD4⁺ CD25⁺ Treg offen-oder nahegelegt wird.

Die regulatorischen Zellen können durch die Expression eines und/oder mehrerer Marker identifeiert und/oder separiert werden. Hierfür können alle dem Fachmann bekannten Marker zur Identifizierung und/oder Separation eingesetzt werden. Bevorzugt werden die Marker 4-1 BB, CD25, CTLA-4 (cytotoxic T lymphocyte antigen-4), GITR (Glucocorticoid-induced TNFRezeptor), FoxP3, IL-10, CD69, CD40L, ICOS, OX40 und TGFbeta, die einzeln und/oder in Kombination eingesetzt werden. Hierfür können auch alle dem Fachmann bekannten Marker zum Ausschluß oder der Depletion von nicht-regulatorischen Zellen in Kombination eingesetzt werden. Besonders bevorzugt ist aber die Verwendung des 4-1BB Rezeptors als Marker von lebenden, aktivierten regulatorischen Zellen. Entweder werden direkt aktivierte regulatorische T-Zellen identifiziert oder separiert oder die Aktivierung wird durch Zugabe von Zellen, Proteinen, Peptiden, Pathogenen oder anderen Stoffen induziert

In einem weiteren Aspekt betrifft die Erfindung die überraschende Lehre, dass insbesondere innerhalb einer Zeitabhängigkeit 4-1BB ein spezifischer Marker für aktivierte Treg-Zellen ist, vor allem in einem Zeitraum von 3 bis 8 Stunden; vor allem bei einer Temperatur von 37°C. Durch die zeitabhängige Beobachtung der Zellen innerhalb von Zeiteinheiten kann in einer bevorzugten Ausführungsform 4-1BB als Marker für die Separation und Isolierung von den o. g. aktivierten Zeiten (aktivierte Treg) gemäß Anspruch 1 dienen-d. h., die Erfindung betrifft in einer bevorzugten Ausführungsform die überraschende Lehre, dass aktivierte regulatorische Th-Zellen identifiziert und/oder separiert werden können, insbesondere wenn eine zeitabhängige Beobachtung durchgeführt wird, innerhalb derer der 4-1 BB Rezeptor zur Isolierung von CD4⁺ CD25⁺ Treg aus Zellengemischen gegenüber CD4⁺ CD25⁻ Th-Zellen verwendet werden kann. Insbesondere die zeitabhängige Beobachtung der Zellen ermöglicht die Verwendung von 4-1 BB als diskriminativen Marker für CD25⁺ Treg im Vergleich zu CD25⁻ T-Zellen. Auch wenn zahlreiche Parameterbestimmungen im Stand der Technik zeitabhängig erfolgen, ist es im Vergleich zum Stand der Technik überraschend, dass in den o. g. bevorzugten Zeiträumen die Ausprägung des Markers so asymmetrisch innerhalb einer Zellkultur auftritt, dass dieser zur Identifizierung oder Separation von aktivierten regulatorischen Th-Zellen (Treg) gegenüber CD4⁺ CD25⁻ Th-Zellen verwendet werden kann. Es schien bisher ausgeschlossen, 4-1BB als spezifischen Marker für CD25 Treg gegenüber CD4⁺ CD25⁻ Th-Zellen zu nutzen. So offenbart beispielsweise die WO 2005/124346 nicht, dass es sich bei 4-1 BB Rezeptoren um eine ganz spezifische Signatur handelt, die sogar eine direkte Isolierung ermöglicht. Die Dauer der Inkubation, insbesondere über eine Dauer von 3 bis 8 Stunden, bevorzugt 4 Stunden, erlaubt die Verwendung von 4-1BB als diskriminativer Marker für Treg gegenüber CD4⁺ CD25⁻ Th-Zellen. Diese Lehre führt in eine andere Richtung als der gesamte Stand der Technik. Durch die Offenbarung der erfindungsgemäßen Lehre kann der durchschnittliche Fachmann diese modifizieren und auf äquivalente Verwendungen erstrecken. Beispielsweise wird er erkennen, dass die Aktivierung auch über einen Zeitraum von 18 Stunden, wenn dem System bestimmte Inhibitoren zugesetzt werden. Wenn dem System beispielsweise ein Zusatz hinzugefügt wird, der verhindert, dass regulatorische T-Zellen aktiviert werden, dann wäre der Zeitraum der Aktivierung länger-als 18 Stunden. Innerhalb dieses Systems würde der Fachmann eine Zeitdauer von über 18 Stunden als äquivalent zu 18 Stunden ohne einen Zusatz, der verhindert, dass regulatorische T-Zellen aktiviert werden, erkennen. Der Fachmann versteht die anmeldungsgemäße Lehre so, dass die Zellen aktiviert werden. Dies gilt beispielsweise für die in vitro Versuche. Dem Fachmann ist aber bekannt, dass in in vivo Systemen einige Zellen immer aktiviert sein können. Beispielsweise würden immer einige Zellen, die beispielsweise an einem rheumatischen Entzündungsherd entnommen würden, aktiviert sein, so dass in einer entsprechenden Probe regulatorisch aktivierte Zellen vorliegen. Dem Fachmann ist auch bekannt, dass beispielsweise direkt aus lymphatischem Gewebe isolierte Treg (z. B. bei Experimenten mit Mauszellen, bei denen die Zellen grundsätzlich aus der Milz präpariert werden) ein geringer Teil der isolierten Treg eine schwache Expression von 4-1 BB aufweist, wohingegen bei aus peripherem Blut stammenden Treg keine 4-1 BB Expression detektierbar ist Der Fachmann erkennt aber die Äquivalenz der in vitro und der in vivo Systeme. Bevorzugt müssen die Treg beispielsweise in vitro aktiviert werden, wobei der Fachmann diese Lehre nicht so auslegen würde, dass dies bei ausgewählten Zellen auch der Fall ist, wenn die Treg direkt aus einem entzündeten Probenmaterial isoliert werden. In einer solchen Situation ist es denkbar, die Zellen ohne Aktivierung direkt anhand ihrer 4-1 BB Expression zu isolieren. Unter der Annahme, dass die Treg direkt am Ort der Entzündung in vivo aktiviert worden sind, ist es bevorzugt, solche Zellen zu isolieren, in vitro zu expandieren und sie z. B. bei Entzündungen mit einhergehender chronischer Autoimmunität zur Therapie zu reinfundieren. Die Erkenntnis der zeitlichen Ausprägung eines diskriminativen Markers insbesondere in einem

Zeitraum von ungefähr oder im wesentlichen 3 bis 8 Stunden nach einer Aktivierung beispielsweise in in vitro Kulturen bzw. eines äquivalenten Zeitraums in in vivo Kulturen, unter der Berücksichtigung, dass hier regelmäßig einige aktivierte Zellen vorlieben, ermöglicht dem Fachmann die Isolierung von Antigenspezifischen CD25⁺ regulatorischen Th-Zellen.

Die getrennten Zellen können in jedem entsprechenden Behältnis - z.B. einer Sammetröhre - aufgefangen und/oder gesammelt werden, welche das Überleben und/oder die Vermehrung der Zellen ermöglicht Verschiedene Medien sind im Handel verfügbar und können je nach der Art der identifizierten und/oder separierten Zellen gebraucht werden. Diese Medien können z.B. DMEM, HBSS, dPBS, RPMI, Iscove's Medium usw. sein, welche häufig mit fötalem Kalbsserum, humanem Serum oder Serumersatzstoffen ergänzt werden.

Die identifizierten und/oder separierten Zellpopulationen können sofort benutzt werden oder nach Isolierung in vitro kultiviert werden. Hiernach können die Zellen eingefroren werden, oder man friert sie vor dem Trennungsverfahren ein. Falls die Zellen für einen längeren Zeitraum gelagert werden, ist es bevorzugt sie bei Temperaturen um -80°C oder in flüssigen Stickstoff einzufrieren, um zu garantieren, dass sie nach dem Auftauen wieder verwenden werden können. Hierbei werden die Zellen normalerweise in DMSO und/oder FCS/HS in Verbindung mit einem Medium, Glucose usw. gelagert Sobald aufgetaut, können die Zellen entweder direkt für therapeutische Zwecke oder in vitro Versuche verwendet werden oder durch die Verwendung von Wachstumsfaktoren, Antigenen, Zellen usw. vermehrt und/oder differenziert werden.

Bevorzugt ist es, die regulaborischen Th-Zellen aus einem Zellgemisch zu identifizieren und zu isolieren. Die regulatorischen Th-Zellen werden aus Zellproben von Säugetieren (Mamalia), aber besonders von Menschen und bevorzugt aus gesunden Probanden und/oder Patienter in vitro identifiziert und/oder separiert. Die Zellproben können zB. aus Blutproben, die Immunzellen enthalten (peritoneale, cerebrale-spinale, pleurale und/oder synoviale Körperflüssigkeit), Spülungsflüssigkeiten von Hohlorganen (Atemwege, Lungen), Homogenaten und Aspirata von Lymphknoten, Milz, Mandeln und/oder anderem lymphatischem Gewebe stammen. Die Zellen können aus Tiergeweben gewonnen werden (z.B. von der Milz oder den Lymphknoten eines Tieres). Die Zellen können auch Teil einer Blutprobe sein, z.B. von einer Versuchperson oder menschlichem Patienten, besonders bevorzugt aus einer peripheren Blutprobe.

In einer weiteren bevorzugten Ausführung, ist vorgesehen, dass aktivierte regulatorische Th-Zellen gemäß Anspruch 1 identifiziert und/oder separiert werden.

Aktivierte regulatorische Th-Zellen sind direkt in der Lage, die Aktivität von T-Zellen zu unterdrücken. In Tiermodellen ist gezeigt worden, dass aktivierte regulatorischen T-Zellen die Entwicklung von Autoimmunerkrankungen, von Transplantatabstoßungsreaktionen und allergischen Reaktionen verhindern können. In Analogie dazu vermutet man, dass aktivierte regulatorische T-Zellen auch beim Menschen die Entstehung von chronischen Immunreaktionen verhindern können. Durch die Verwendung des 4-1 BB Markers werden aktivierte regulatorische Th-Zellen spezifisch identifiziert und/oder separiert. Bevorzugt werden zuvor in vitro aktivierte regutatonsche Th-Zellen mit dem Phänotyp CD4+ CD25+ FoxP3+ identifiziert und/oder separiert werden. Die Aktivierung erfolgt hierbei bevorzugt mittels Antigenen, Antikörpern, Peptiden, Proteinen, chemischen Verbindungen (oder Gemischen solcher Stoffe) oder Pathogenen oder Zellen. Eine weitere besondere Ausführungsform der Erfindung ist es, lebende regulatorische Th-Zellen direkt ex vivo gemäß Anspruch 1 zu identifizieren und/oder zu isolieren.

Durch die Verwendung des 4-1 BB Markers und dem entsprechenden 4-1 BB Antigen können bevorzugt aktivierte regulatorischen Th-Zellen identifiziert und/oder separiert werden, was eine eine bevorzugte Verwendung der Erfindung, zur Indentifizierung und Isolierung Antigen-spezifischer regulatorische Th-Zellen gemäß Anspruch 1 erlaubt.

Nach der Gewinnung eines Zellgemisches aus einem Patienten und/oder Probanten, kann diese mit bestimmten Antigenen stimuliert werden. Hierzu können Antikörper, Peptide, Proteine, chemische Verbindungen (oder Gemische solcher Stoffe) oder Pathogene oder Zellen verwendet werden. Nach einer bestimmten Zeit der Aktivierung werden durch die Verwendung des 4-1BB Markers die regulatorischen Th-Zellen identifiziert und/oder separiert, Hierbei werden bevorzugt Antigen-spezifische regulatorische Th-Zellen identifiziert und/oder separiert werden.

Die Zellen die nach der Erfindung separiert werden stammen im Allgemeinen von einer in vivo Quelle ab und reflektieren daher den immunologischen Status des Spenders in Bezug auf die Anzahl, den Standort und die T Zellantigenrezeptorspezifität der Treg Zellen wieder. Diese Informationen können bei der diagnostischen Prüfungen verwendet werden, die sich auf immunologische Störungen beziehen, z.B. Krebs bezogene Immunosuppression, autoimmune Störungen; atopische Zustände, usw.

Des Weiteren können Th-Zellen des Zielpatienten in vitro Zellen, Zellextrakten, separierten Antigenen oder Proteinen des Organspenders vor oder nach der Transplantation ausgesetzt werden. Hiernach würden die aktiven regulatorischen Th-zellen nach einer angebrachten Aktivierungszeit anhand des 4-1 BB Markers identifiziert und/oder separiert werden.

Die Zellen können durch alle dem Fachmann bekannten Techniken identifiziert und/oder separiert werden. Zur Identifikation der Zellen eignen sich bevorzugt die Methoden magnetische Zellsortierung (MACS), fluoreszenzaktivierte Zellsortierung (FACS), ELISA, PCR und/oder alle dem Fachmann bekannte Fluoreszenzmikroskope. Besonders bevorzugt eignet sich die Durchflußzytometrie (FACS). Für die Separation der Zellen eignen sich bevorzugt die Zelttrennungssysteme, durch die Zellen mit Hilfe eines Magnetfeldes (MACS) oder durch Durchflußzytrometrie (FACS) sortiert werden. Bevorzugt ist es, dass die Expression des 4-1 BB Rezeptors nach einer Stimulation gemessen wird.

Die regulatorischen Th-Zellen werden durch dem Fachmann bekannte Stoffe stimuliert. Nach 3h bis 8h Stimulation wird die Expression des 4-1 BB Rezeptors gemessen. Bevorzugt wird die Expression nach 4h einer Stimulation gemessen.
Der bevorzugte optimale Stimulationszeitpunkt ist der Zeitpunkt an dem möglichst viele der aktivierten, regulatorischen T-Zellen, aber möglichst wenige der nicht-regulatorischen Zellen 4-1B B exprimieren. Dieser Zeitpunkt kann vom Fachmann durch eine Analyse der 4-1 BB Expression zu verschiedenen Zeitpunkten nach Stimulation ermittelt werden.

In einer weiteren bevorzugten Ausführungsform gemäß Anspruch 6, ist vorgesehen, dass die regulatorischen Th-Zellen nach einer Stimulation der Zellen im Zellgemisch identifiziert und/oder separiert werden.

Des Weiteren ist eine bevorzugte Verwendung der Erfindung, dass die Zellen durch Antigene, Proteine, Peptide, chemische Stoffe, Zellen, Wachstumsfaktoren, Antikörper und/oder Liganden stimuliert werden.

Die Kultur kann zudem geeignete Wachstumsfaktoren enthalten. Wachstumsfaktoren sind definiert als Moleküle, die das Überleben, Wachstum und/oder die Differenzierung einer Zelle entweder in Kultur oder im intakten Gewebe durch bestimmte Wirkungen auf einen transmembrane Rezeptor fördem. Wachstumsfaktoren schließen Polypeptide und nicht-polypeptide Faktoren ein. Bestimmte Wachstumsfaktoren, die verwendet werden können, die separierte und/oder verwendete Zelle zu kultivieren, schließen die Interieukine, z.B. IL-1, IL-2, IL-3, IL-4, IL-5, IL-6, IL-7, IL-8, IL-8, IL-10, IL-11, IL-12, IL-13, IL-14, IL-15, IL-16, IL-17, IL -18 usw. ein; Antigene, zB. Peptidantigene, Proteinantigene wie z.B. Alloantigene vorzugsweise in Verbindung mit Antigenen zeigenden Zellen, Lectine, z.B. ConA, [Alpha]-CD3, LPs, usw.

Die Kultur kann auch Antikörper oder spezifische Liganden (gereinigte Liganden, FC Verschmelzungsproteinen oder andere, rekombinante Formen des Leucinzipper) für Zelloberflächenrezeptoren enthalten, die die Treg Aktivität stimulieren oder hemmen können. Im Medium können auch mAb oder Liganden, die TNFR oder andere Ko-stimulierende Moleküle auf Treg binden und die Treg Aktivität stimulieren und/oder steigem, Treg Aktivität außer Kraft setzen (und eine Vermehrung herbeiführen), oder die Apoptosis der Treg stimulieren enthalten sein. Normalerweise ist es gewollt, dass die spezifischen Kulturbedingungen einen besonderen Zweck dienen, z.B. die Erhaltung der Treg Zellaktivität usw.

Bevorzugt ist es, dass Antikörper zur Detektion des 4-1 BB Rezeptors gemäß der erfindungsgemäßen Verwendung des 4-1BB-Rezeptors als diskriminativer Marker verwendet werden.

Die 4-1 BB Antikörper werden einer Zellsuspension hinzugefügt und für einen ausreichenden Zeitraum inkubiert, um die verfügbaren Zelloberflächenantigene zu binden. Die Inkubation beträgt normalerweise mindestens etwa 5 Minuten bis zu 18h. Die Expression wird nach 3h bis 8h einer Stimulation gemessen. Der optimale Inkubationszeitraum ist der, innerhalb dessen möglichst viele der aktivierten, regulatorischen T-Zellen, aber möglichst wenige der nicht-regulatorischen Zellen 4-1 B markiert werden. Dieser Zeitrahmen kann vom Fachmann durch eine Analyse verschieden langer Inkubationszeiten während oder nach der Stimulation ermittelt werden.
Es ist wünschenswert, eine ausreichende Konzentration von Antikörpern in der Reaktionsmischung zu haben, so dass die Effizienz der Trennung nicht vom Mangel an Antikörper eingegrenzt wird. Die entsprechende Konzentration kann auch durch Titration bestimmt werden.
Das Mittel, worin die Zellen getrennt werden, kann jedes Mittel sein, das die Aktivität der Zellen erhält. Ein bevorzugtes Mittel ist eine mit Phosphat gepuffertes Salzlösung, die 0,1 bis 0,5% BSA oder in gleichen Anteilen autologes, gepooltes Serum oder Serumersatzstoffe enthält. Verschiedene Medien sind im Handel verfügbar und können abhängig vom Zellentyp und des Experimentes verwendet werden. Verfügbare Medien sind Dulbecco's modifiziertes Eagle's Medium (dMEM), Hank's Basic Salt Solution (HBSS) ein Dulbecco's Phosphat gepufferte Salzlösung (dPBS), RPMI, Iscove's Medium, PBS mit 5 mM EDTA usw. häufig ergänzte durch fötalem Kalbsserum BSA, HSA usw.

Die gekennzeichneten Zellen werden dann abhängig von der Expression von 4-1BB getrennt. Die getrennten Zellen können in jedem entsprechenden Mittel gesammelt werden, das die Lebensfähigkeit der Zellen erhält Normalerweise werden sie in einer Sammelröhre mit etwas Serum gesammelt Verschiedene Medien sind im Handel verfügbar und können je nach Art der identifizierten und/oder separierten Zellen verwendet werden, zB. dMEM, HBSS einschließend, dPBS, RPMI, Iscove's Medium usw., häufig ergänzt mit fötalem Kalbsserum, autologen oder gepoolten Serum oder Serumersatzstoffen.

Des Weiteren ist bevorzugt, dass die Antigene zur Detektion des 4-1 BB Rezeptors mit einem fluoreszierenden Stoff oder direkt mit magnetischen Mikropartikeln gekoppelt sind.

Für die Durchflußzytometrie oder FACS (Fluorescence activated cell sorting) und die magnetische Zellsortierung oder MACS (Magnetic activated cell sorting) werden Antikörper eingesetzt, die mit Fluoreszenunarkem oder magnetischen Mikropartikeln gekoppelt sein können, die dem Fachmann bekannt sind, wie z.B FITC, Phycoerythrin (PE), Allophycocyanin (APC), Cascade yellow and Peridinin Chlorophyll Protein (PerCP). Die Antikörper können auch an Haptene gekoppelt sein und dann mit Hapten-spezifischen Sekundärantikörpern detektiert zu werden. Eine Kombination von Antikörpern kann benutzt werden, um verschiedene Zellen mit unterschiedlichen Eigenschaften zu detektieren, analysieren und/oder zu isolieren, auf der Basis von Markem, wie z.B. sekretorische Proteine oder charakteristische Oberflächenmoleküle. Besonders können fluoreszenzmarkierte Antikörper für FoxP3, CD25 und/oder CD4 Marker eingesetzt werden. Besonders bevorzugt können fluoreszenzmarkierte oder mit magnetischen Mikropartikeln gekoppelte 4-1BB-Antikörper eingesetzt werden. Magnetische Mikropartikel können hierbei direkt oder indirekt verwendet werden (Hapten z.B. Biotin und Anti-Hapten Mikropartikel oder Fluorochrome, z.B. PE oder APC und Anti-Fluororchrom Mikropartikel).

In einer weiteren bevorzugten Ausführungsform, ist vorgesehen, dass die regulatorischen Th-Zellen aus Blut, periphere mononukleären Blutzellen (PBMC), Körpergewebe oder Zellen der Gewebeflüssigkeit in vitro direkt identifiziert und/oder separiert bzw. isoliert werden.

Normalerweise werden die regulatorischen Th-Zellen aus Zellproben von Säugetieren (Mamalia), aber besonders von Menschen und bevorzugt aus Versuchperson und/oder Patienten identifiziert und/oder separiert Die regulatorischen Th-Zellen können z.B. aus Blutproben, die Immunzellen enthalten (peritoneale, cerebrale-spinale, pleurale und/oder synoviale Körperflüssigkeit), Spülungsflussigkeiten von Hohlorganen (Atemwege, Lungen), Homogenaten und Aspirata von Lymphknoten, Milz, Mandeln und/oder anderem lymphatischem Gewebe stammen. Die regulatorischen Th-Zellen können auch aus Tiergeweben gewonnen werden (z.B. von der Milz oder den Lymphknoten eines Tieres). Die Th-Zellen können auch Teil einer Blutprobe sein, z.B. von einer Versuchperson oder menschlichem Patienten, besonders bevorzugt aus peripheren mononukleären Blutzellen (PBMC), Körpergewebe oder Zellen der Gewebeflüssigkeit

Die Erfindung sieht die Verwendung eines Kits vor, umfassend einen Antikörper zur Detektion des 4-1BB Rezeptors zur Identifikation und/oder Separation einer regulatorischen Th-Zelle gemäß der erfindungsgemäßen Verwendung des 4-1BB-Rezeptors als diskriminativer Marker.

Bevorzugt ist es, dass im Kit der 4-1 BB Rezeptor Antikörper mit einem fluoreszierenden Stoff, mit einem Hapten oder mit magnetischen Mikropartikeln gekoppelt ist.

Für die Identifikation und/oder Separation der aktivierten, lebenden regulatorischen Th-Zellen wird ein 4-1 BB Rezeptor Antikörper bereitgestellt, der an einem Fluoreszenzmarker, einem Hapten oder magnetischen Mikropartiklen gekoppelt ist. Fluoreszenzmarker sind dem Fachmann bekannt, wie z.B FITC, Phycoerythrin (PE), Allophycocyanin (APC), Cascade yellow and Peridinin Chlorophyll Protein (PerCP).

Des Weiteren ist eine bevorzugte Eigenschaft des Kits, dass aktivierte regulatorische Th-Zellen identifiziert und/oder separiert werden.

Durch die Verwendung des Kits, umfassend einen 4-1 BB Rezeptor Antikörper gekoppelt mit einem Fluoreszenzmarker, Haptenen oder magnetischen Mikropartikeln können regulatorische Th-Zellen identifiziert und/oder separiert werden. Durch die Anwendung des 4-1 BB Rezeptor Antikörpers können bevorzugt aktivierte regulatorische Th-Zellen identifiziert und/oder separiert werden. Besonders bevorzugt werden lebende, aktivierte regulatorische Th-Zellen identifiziert und/oder separiert.

In dieser Ausführung kann die Methode der Erfindung z.B. genutzt werden, um regulatorische Th-Zellen ex vivo aus einer Blutprobe (oder aus einem Blutderivat) oder aus Zellgemischdenvaten von lymphatischen Organen oder Tumoren von Säugetieren identifiziert und/oder separiert werden.

Die separierten regulatorischen Th-Zellen können vor und/oder nach dem Klonieren und/oder Vermehrung und/oder in Zellgemischen angereichert in und/oder als pharmazeutisches Mittel in der Therapie oder Vorbeugung von Krankheiten genutzt werden. Aus den separierten regulatorischen T-Zellen können ausserdem die kodierenden Gensequenzen des TCR (T-Zellrezptor) isoliert werden und für weitere therapeutische Zwecke wie zB für Krebstherapien genutzt werden. Außerdem können die in der genannten Form vorliegenden regulatorischen Th-Zellen in weiteren Untersuchungen und/oder Analysen eingesetzt werden. Das pharmazeutische Mittel kann für die Behandlung und/oder Vorbeugung von Krankheiten in Säugetieren verwendet werden, was eine Verabreichung einer pharmazeutisch wirksamen Menge des pharmazeutischen Mittels in das Säugetier einschließen kann.

Die Krankheit kann irgendeine Krankheit sein, die durch die Gegenwart von einer separierten Zelle und/oder durch die Konzentrationserhöhung der betreffenden Zellen in/an der betreffenden Stelle, oder im gesamten Säugetierprobanden und/oder Patienten behandelt und/oder vorgebeugt werden kann. Zum Beispiel kann die Zelle eine regulatorische Th-Zelle sein und die behandelte und/oder präventiv behandelte Krankheit kann eine Autoimmunkrankheit, eine infektiöse Krankheit, eine Allergie, Transplantat versus Wirtskrankheit (oder allogene Transplantatabstoßung) und/oder irgendeine andere durch Hypersensitivität eingeleitete Krankheit sein.

Krankheiten, wofür die in der Erfindung dargelegte Verwendung besonders geeignet ist, sind solche, die durch und/oder während einer fehlenden Regulation der Immunantwort entstehen. Diese Krankheiten können Transplantatabstoßungen sein, allergische Zustände, bestimmte infektiöse Krankheiten und/oder Autoimmunkrankheiten sein.

Im Zusammenhang mit Autoimmunkrankheiten ist es bekannt, dass das Immunsystem körpereigene Strukturen attackiert, wie zB. bei rheumatischer Arthritis, Insulin abhängiger Diabetes mellitus (IDDM), Multiple Sklerose (MS), Atherosklerose, Psoriasis, allogener Stammzellentransplantation, Organtransplantation. Eine Therapie mit spezifischen regulatorischen T-Zellen ist hier vorteilhaft. Bei Tumorerkrankungen sollen regulatorische T-Zellen spezifisch für Tumorantigene eliminiert werden, so dass das Immunsystem effiziente anti-Tumor Immunantworten initieren kann.

Die beschriebene Zusammensetzung und/oder das beschriebene pharmazeutische Mittel Könnten zur Behandlung von Krankheiten, die mit einer Defizienz der zellulären Immunität verbunden sind, zum Beispiel bei dem Defekt nach ICDIO-code: D.84.4 verwendet werden. Hierbei kann es sich um Sepsiserkrankungen handeln, um Entzündungs-reaktionen und Fieber, um AutoimmunErkrankungen und Erkrankungen der Störung der Zellteilung wie zum Beispiel Krebs. Entzündungen sind die vom Bindegewebe und den Blutgefäßen getragene Reaktion des Organismus auf einen äußeren oder innerlich ausgelösten Entzündungsreiz mit dem Zweck, diesen zu beseitigen oder zu inaktivieren und die reizbedingte Gewebsschädigung zu reparieren. Auslösend wirken mechanische Reize (Fremdkörper, Druck, Verletzung) und andere physikalische Faktoren (ionisierende Strahlen, UV-Licht, Wärme, Kälte), chemische Stoffe (Laugen, Säuren, Schwermetalle, bakterielle Toxine, Allergene und Immunkomplexe) sowie Erreger(Mikroorganismen, Würmer, Insekten) bzw. krankhafte Stoffwechselprodukte, entgleiste Enzyme, bösartige Tumoren. Das Geschehen beginnt mit einer kurzen Arteriolenverengung (durch Adrenalinwirkung) mit Mangeldurchblutung und Gewebsalteration, gefolgt von der Eritwicklung der klassischen örtlichen Entzündungszeichen (Kardinalsymptome; nach GALEN und CELSUS), das heißt von Rötung (= Rubor, Gefäßerweiterung durch Histamin), Wärme (= Calor, durch örtliche Stoffwechselsteigerung), Schwellung (= Tumor; durch Austritt eiweißreicher Flüssigkeit aus den - unter anderem durch Histamin - veränderten Gefäßwänden, unterstützt durch die verlangsamte Blutzirkulation im Sinne der Prästase bis Stase), Schmerz (= Dolor, als Folge der erhöhten GewebsSpannung und schmerzauslösender Entzündungs-produkte, zum Beispiel Bradykinin) und Funktionsstörung (= Functio laesa). Der Vorgang wird ergänzt durch Störung des Elektrolythaushaltes (Transmineralisation), Einwanderung neutrophiler Granulozyten und Monozyten durch die Gefäßwände (s.a. Leukotaxis), letzteres mit dem Zweck, den Entzündungsreiz und geschädigte bis neukrotische Zellen zu beseitigen (Phagozytose); ferner wandern LymphozytenEffektorzellen ein, die zur Bildung spezifischer Antikörper gegen den Entzündungsreiz führen (Immunreaktion), sowie Eosinophile (in der Heilungsphase bzw. - sehr frühzeitig - bei allergisch-hyperergischem Geschehen). Durch die bei der Reaktion erfolgende Aktivierung des Komplementsystems werden Bruchstücke (C3a und C5a) dieses Systems frei, die - wie das Histamin und Bradykinin - als Mediatoren der Entzündung wirken, und zwar im Sinne der Anregung der Chemotaxis der zitierten Blutzellen; ferner wird die Blutgerinnung aktiviert. In der Folge tritt eine Schädigung (Dystrophie und Koagulations-nekrose) des zugeordneten Organparenchyms ein. Der Gesamt-Organismus reagiert je nach Intensität und Art der Entzündung mit Fieber, Stress (s.a. Adaptationssyndrom), Leuko-zytose und Veränderungen in der Zusammensetzung der Plasmaproteine (Akute-Phase-Reaktion), die zu einer beschleunigten Blutkörperchensenkungsreaktion führen. Bevorzugte Entzündungen im Sinne der Erfindung sind die eitrige, die exudative, die fibrinöse, die gangräneszierende, die granulomatöse, die hämorrhagische, die katarrhalische, die nekrotisierende, die proliferative oder produktive, die pseudomembranöse, die seröse, die spezifischen und/oder die ulzeröse Entzündungen.

Autoimmunerkrankungen sind Krankheiten, die zum Teil auf die Bildung von Autoantikörpern und deren schädigende Einwirkung auf den Gesamtorganismus bzw. Organsysteme, das heißt auf Autoaggression zurückzuführen sind. Andererseits kann es sich bei Autoimmunerkrankungen auch um Krankheiten halten, bei denen T-Zellen eine herausragende Rolle bei der Pathogenese bzw. Initiation innehaben. Eine Klassifikation ist als organspezifische, intermediäre und/oder systemische Autoimmunerkrankung möglich. Bevorzugte organspezifische Autoimmunerkrankungen sind HASHIMOTO Thyreoiditis, primäres Myxödem, Thyreotoxikose (BASEDOW Krankheit), perniziöse Anämie, ADDISON Krankheit, Myasthenia gravis und/oder juveniler Diabetes mellitus. Bevorzugte intermediäre Autoimmunkrankheiten sind GOODPASTURE Syndrom, autoimmune hämolytische Anämie, autoimmune Leukopenie, idiopathische Thrombo-zytopenie, Pemphigus vulgaris, sympathische Ophthalmie, primäre biliäre Zirrhose, Autoimmunhepatitis, Colitis ulcerosa und/oder SJÖGREN Syndrom. Bevorzugte systemische Autoimmunkrankheiten sind rheumatoide Arthritis, rheumatisches Fieber, systemischer Lupus erythematodes, Dermatomyositis/Polymyositis, progressive systemische Sklerose, WEGENER Granulomatose, Panarteriitis nodosa und/oder Hyper-sensitivitätsangiitis. Typische Autoimmunkrankheiten sind Thyreotoxikose, Schilddrüsenbedingtes Myxödem, HASHIMOTO Thyreoiditis, generalisierte Endokrinopathie, perniziöse Anämie, chronische Gastritis Typ A, Krankheiten einzelner oder aller korpuskularen Elemente des Blutes (zum Beispiel autoimmunhämolytische Anämie, idiopath. Thrombozytopenie bzw. -pathie; idiopath. Leukopenie bzw. Agranulozytose), Pemphigus vulgaris und Pemphigoid, sympathische Ophthalmie und manche Uveitis-Formen, primär biliäre Leberzirrhose und chronisch aggressive Autoimmunhepatitis, Diabetes meUitus Typ I, CROHN Krankheit und Colitis ulcerosa, SJÖGREN Syndrom, ADDISON Krankheit, Lupus erythematodes disseminatus und als diskoide Form dieser Krankheit, als Dermatomyositis und Slderodermie, rheumatoide Arthritis (= primär-chronische Polyarthritis), Antigkomerulusbasalmembran-Nephritis. Grundlage sind eine aggressive Immunreaktion infolge Zusammenbruchs der Immuntoleranz gegenüber Selbst-Derterminanten und ein Übergewicht von inflammatorischen T-Helfer-zellen. Ferner ist die Bildung von Autoantigenen möglich, zum Beispiel durch Verbindung von Wirtsproteinen mit Haptenen (zum Beispiel Armeimittel), durch ontogenetisches Gewebe, das sich erst nach Entwicklung der Selbsttoleranz entwickelt und für durch Änderungen der Konformation der Proteine demaskierte Proteinkomponenten im Zusammenhang zum Beispiel mit Infektion durch Viren oder Bakterien; ferner für im Zusammenhang mit Neoplasien entstandene neue Proteine.

Sepsiserkrankungen sind Erkrankungen infolge dauernden oder periodischen Eindringens von pathogenen Bakterien und/oder deren Giften aus einem Krankheitsherd und deren Ausbreitung auf dem Lymph-Blut-Weg zur allgemein beziehungsweise lokalen Infektion.
Sepsis sind bevorzugt Wundsepsis (Phlegmone, Thrombophlebitis, Lymphangitis), Puerperalsepsis (bei Puerperalfieber), otogene Sepsis (bei Otitis media), tonsillogene Sepsis (bei Angina, Peritonsillitis), cholan-gitische Sepsis (bei eitriger Cholezystzitis, Cholangitis), pylephlebitische Sepsis (bei Pylephtebitis) Nabelsepsis (bei Omphalitis etc.), Urosepsis sowie bei Zahngranulom. Die Sepsis kann akut bis hochakut (foudroyant), subakut (z. Beispiel als Endocarditits lenta) oder chronisch erfolgen, aber selbstverständlich auch als Neugeborenensepsis.
Sepsis sind also alle pathogenen Veränderungen eines Patienten, die mit intermitterierendem Fieber und Schüttelfrost verbunden sein können sowie mit Milztumor, mit toxischen Reaktionen beziehungsweise Schäden des Knochenmarks beziehungsweise des Blutes (polynukleäre Leukozytose, Anämie, Hämolyse, Thrombozytopenie) oder aber mit pathogenen Reaktionen am Herzen und der Gefäßnerven (Tachykardie, Zentralisation des Kreislaufs, Ödeme, Oligurie; evtl. Schock) beziehungsweise des Verdauungstraktes (trockene, belegte Zunge, Durchfälle) oder aber mit Septikopyämie (Pyämie mit Bildung septischer Infarkte und metastatischer Abszess).

Bevorzugte Erkrankungen, die mit einer Defizien des zellulären Immunsystems verbunden sind, sind weiterhin:
AIDS, Akne, Albuminurie (Proteinurie), Alkoholentzugs-syndrom, Allergien, Alopezie (Haarausfall), ALS (Amyotrophe Lateralsklerose), Alzheimer Erkrankung, AMD (Altersbedingte Makuladegeneration), Anämie, Angststörungen, Anthrax (Milzbrand), Aortensklerose, arterielle Verschlusskrankheit, Arterienverkalkung, Arterienverschluss, Arteriitis temporalis, Arteriosklerose, Arteriovenöse Fisteln, Arthritis, Arthrose, Asthma, Ateminsuffizienz, Autoimmunerkrankung, AV-Block, Azidose, Bandscheibenvorfall, Bauchfellentzündung, Bauchspeicheldrüsenkrebs, Becker Muskeldystrophie. Benigne Prostata Hyperplasie (BPH), Blasenkarzinom, Bluterkrankheit
(Hämophilie), Bronchialkarzinom, Brustkrebs, BSE, Budd-Chiari-Syndrom, Bulimia nervosa, Bursitis (Schleimbeutelentzündung), Byler-Syndrom, Bypass, Chlamyderf-Infektion, Chronische Schmerzen, Cirrhose, Commotio cerebri (Gehirnerschütterung), Creutzfeld-Jakob-Erkrankung, Darmkarzinom, Darmkrebs, Darmtuberkulose, Depression, Diabetes insipidus, Diabetes mellitus, Diabetes mellitus juvenilis, Diabetische Retinopathie, Duchenne-Muskeldystrophie, Duodenafkarzinom, Dystrophia musculorum progressiva, Dystrophie, Ebola; Ekzem, Erektile Dysfunktion, Fettsucht, Fibröse, Gebärmutterhalskrebs, Gebärmutterkrebs, Gehimblutung, Gehimentzündung, Haarausfall, Halbseitenlähmung, Hämolytische Anämie, Hämophilie, Haustierallergie (Tierhaarallergie), Hautkrebs, Herpes zoster, Herzinfarkt, Herzinsuffizienz, Herzklappenentzündung, Himmetastase, Himschlag, Himtumor, Hodenkrebs, Ischämie, Kahler-Krankheit (Plasmozytom), Kinderlähmung (Poliomyelitis), Knochenschwund, Kontaktekzem, Lähmung, Leberzinhose, Leukämie, Lungenfibrose, Lungenkrebs, Lungenödem, Lymphdrüsenkrebs (Morbus Hodgkin), Lymphogranulomatose, Lymphom, Lyssa, Magenkarzinom, Mammakarzinom, Meningitis, Milzbrand, Mukoviszidose (zystrische Fibröse), Multiple Sklerose (MS), Myokardinfarkt, Neurodermitis, Neurofibromatose, Neuronale Tumoren, Nierenkrebs (Nierenzellkarzinom), Osteoporose, Pankreaskarzinom, Pneumonie, Polyneuropathien, Potenz-Störungen, Progressive Systemische Sklerose (PSS), Prostatakrebs, Quaddelauschlag (Urtikaria), Querschnittssyndrom, traumatisches, Rektumkarzinom, Rippenfellentzündung, Schädel-Him-Trauma, Scheidenkrebs (Vaginalkarzinom), Sinusitis, Speiseröhrenkrebs, Tremor, Tuberkulose, Tumorschmerz, Vaginalkarzinom, Verbrennung -Verbrühung, Vergiftungen, Virale Meningitis, Wechseljahre, Weichteilsarkom, Weichteiltumor, Zerebrale Durchblutungsstörungen und/oder ZNS-Tumore.

Die Krebserkrankung oder der Tumor, die/der behandelt oder verhindert werden könnte ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindes-alter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.
Insbesondere kann es sich bei den Tumoren um folgende Krebsarten handeln: Adenokarzinom der Brust. der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basatzeften-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzeilen-Leukämie, T-Zellen-Leukämie, chronische T-Zellen-Leukämie, HTLV-II-assoziierte Leukämie, akut lymphozytische Leukämie, chronisch-tymphozythische Leukämie, Mastzell-Leukämie und myeloische Leukämie); maligne Histiocytose, Hodgkin-Krankheit, non-Hodgkin-Lymphom, solitärer Plasmazelltumor; Reti-culoendotheliose, Chondroblastom; Chondrom, Chondrosarkom; Fibrom; Fibrosarkom; Riesenzell-Tumore; Histiocytom; Lipom; Uposarkom; Leukosarkom; Mesotheliom; Myxom; Myxosarkom; Osteom; Osteosarkom; Ewing-Sarkom; Synoviom; Adenofibrom; Adenolymphom; Karzinosarkom, Chordom, Craniopharyngiom, Dysgerminom, Hamartom; Mesenchymom: Mesonephrom, Myosarkom, Ameloblastom, Cementom; Odontom; Teratom; Thymom, Chorio-blastom; Adenokarzinom, Adenom; Cholangiom; Cholesteatom; Cylindrom; Cystadenocarcinom, Cystadenom; Granulosa-Zelltumor, Gynadroblastom; Hidradenom; Inselzelltumor, Leydig-Zelltumor, Papilom; Sertoli-Zeil-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Himanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

Die Krebserkränkung oder der Tumor, die/der behandelt oder verhindert werden könnte, ist ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome. Urogenitaltumoren umfassend Tumoren der Nieren, der Hamleiter, der Blase, der Prostata, der Hamröhre, des Penis und der Hoden, gynäkologische Tumoren umfassend Tumoren des Zervix, der Vagina, der Vulva, Korpuskarzirnom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syn-drom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Trans-plantations-bezogene Malignitäten, metastasierte Tumoren umfassend Gehimmetastasen, Lungenmetastasen, Lebermetastasen, Knochenmetastasen, pleurale und perikardiale Metastasen und maligne Aszites.
Die Krebserkrankung oder der Tumor, die/der behandelt oder verhindert werden könnte, ist ausgewählt aus der Gruppe umfassend Krebserkrankungen oder Tumorerkrankungen der Mammakarzinome, der Gastrointes-tinaltumore, einschließlich Kolonkarzinome, Magenkarzinome, Pankreaskarzinome, Dickdarmkrebs. Dünndarmkrebs, der Ovarialkarzinome, der Zervikalkarzinome, Lungenkrebs, Prostatakrebs, Nierenzellkarzinome und/oder Lebermetastasen.

Immunopathologische Reaktionen verursachen den Funktionsvertust eines bestimmten Gewebes und können Gewebe zerstören. Die erfindungsgemäße Verwendung erlaubt es aktive regulatorische Th- Zellen von Patienten mit diesen Krankheiten zu isolieren. Die gewebeschädigende Immunantwort kann behandelt werden. Bei Tumorerkrankungen soll die beschriebene Verwendung eine spezifische Elimierung von Tumorspezifischen Treg in vitro oder ex vivo ermöglichen. Falls die regulatorischen Th-Zellen in einer zu geringen Anzahl vorliegen, können sie separiert und angereichert werden und/oder in Zellkulturen vermehrt werden. In beiden Fällen könnte der Patient mit der angereicherten und/oder vermehrten und somit erhöhten Anzahl von regulatorischen Th-Zellen behandelt werden, wodurch eine Unterdrückung der immunopathologischen Antwort und somit eine Verbesserung und/oder Heilung der Krankheit erwartet würde. Autoimmunkrankheiten, wofür der 4-1 BB Rezeptors als Marker zur Identifikation und/oder Isolierung und folgender Anreicherung von aktiven regulatorischen Th-Zellen verwendet werden könnte und ein erfolgreiche Therapie erwartet würde, beinhalten, aber nicht nur auf diese beschränkt. Diabetes mellitus (IDDM), Multiple Sklerose (MS), Atherosklerose (AS), Psoriasis, Darmentzündungen, autoimmunbedingte haemolytische Anämie, Sjogrenssyndrom, autoimmunbedingte Thyroiditis und systemtische Lupus Erythematosis.

Dem Fachmann ist bekannt, dass regulatorische Th-Zellen im Zusammenhang mit Transplantationen fähig sind eine Immunantworten zu unterdrücken, die zu einer Transplantatabstoßung führen könnte. Die Behandlung eines Patienten und/oder Probanden mit zunächst isolierten und dann angereicherten spezifisch aktivierten regulatorischen Th-Zellen könnte eine Transplantatabstoßung vorbeugen und/oder verhindern. Transplantatabstoßungen werden durch eine Immunantwort des Transplantatakzeptors ausgelöst Hierbei bekämpfen die Immunzellen des Akzeptors das körperfremde Gewebe. In dieser Anwendungsform würden Th-Zellen des Zielpatienten in vitro Zellen, Zellextrakten, isolierten Antigenen oder Proteinen des Organspenders vor oder nach der Transplantation ausgesetzt werden. Hiernach würden die aktiven regulatorischen Th-zellen nach einer angebrachten Vermehrungszeit anhand des 4-1 BB Markers identifiziert und/oder separiert werden. Die identifizierten und/oder separierten aktiven regulatorischen Th-Zellen würden dann dem Transplantatakzeptor wieder zugeführt werden. Es wird so erreicht, dass die nach dieser Methode behandelten Zellen die Immunantwort gegen das Transplantat unterbinden und die Transplantatabstoßung verzögert oder verhindert wird. Die Verwendung des 4-1 BB Markers könnte unter anderen bei Allogentransplantaten und/oder Heterotransplantaten, wie zB. bei Herz-, Lungen-, Darm-, Komea-, Nieren- und Knochenmarktransplantationen eingesetzt werden.

Im Falle einer Allergie, würden regulatorische Th-Zellen entweder vor der polyklonalen Aktivierung der T-Zellen oder nach der Kultivierung mit spezifischen Allergenen identifiziert und/oder separiert werden. Die identifizierten und/oder separierten Zellen würden dann nach einer eventuellen Vermehrung dem Patienten zurückgegeben. Es wird nun erwartet, dass die so behandelten regulatorischen Th-Zellen die Immunantwort unterdrücken und sich die zu den verwendeten Antigenen korrelierende Symptomatik verbessert

Die Behandlung von Allergien anhand der Identifizierung und/oder Isolierung von aktiven regulatorische Th-Zellen mit dem Marker 4-1 BB und der beschriebenen Methode würde sich unter anderen bei Hautauschlägen, atopischer Dermatitis. Asthma, allergischer Rhinitis, Insektengiften und Lebensmittelallergie wie z.B. gegen Gluten. Milchprodukten, Nüssen und/oder Fischantigenen eignen.

Bei infektiösen Erkrankungen ist es von Bedeutung die Immunantwort zu stärken als sie zu schwächen. In bestimmten Krankheiten ist es von belang die Immunantwort zu modulieren um den Krankheitsverlauf zu verändem. Solche Fälle der Modulation treten bei Krankheiten auf, wo durch die Infektion immunopathologische Reaktionen auftreten, die Gewebe zerstören und bei denen wo die Immunantwort den Körper nicht schützt. Durch die Identifizierung und/oder Isolierung von aktiven regulatorischen Th-Zellen könnte die Immunantwort unterdrückt werden und immunopathologische Schäden gelindert und/oder verhindert werden.

Eine weitere Alternative, wäre die Behandlung mit aus einem Patienten isolierten regulatorischen Th-Zellen, die bestimmten Antigenen ausgesetzt wurden, um regulatorische Th-Zellen zu erhalten, die die Immunantwort in eine definierte, bestimmte Richtung umschalten könnten.

Gene können in die Zellen vor Kultur oder Transplantation für eine Vielfalt von Zwecken eingeführt werden, z.B. um die Empfänglichkeit einer Infektion zu verhindem oder zu reduzieren, Gene zu ersetzen, die einen Verlust einer Funktionsmutation unterliegen, die Fähigkeit der Treg zu steigem, um Th-Zellen zu hemmen usw. Des Weiteren können Vektoren eingeführt werden die, antisense mRNA oder Ribozyme eprimieren, wodurch die Expression eines unerwünschten Gens blockiert wird. Andere Methoden einer Gentherapie sind die Einführung von Medikamentenwiderstandsgenen, zum Beispiel das Medikamentenwiderstandsgen (MDR) oder Antiapoptosis Genen, wie bcl -2. Weiterhin können Techniken angewendet werden, die dem Fachmann bekannt sind, um Zielzellen zu transfizieren, z.B. durch Elektroporation, Kalzium gefällte DNA, Verschmelzung, Transfektion oder Lipofektion. Die besondere Art, in der die DNA eingeführt wird, ist für die Verwendung der Erfindung nicht entscheidend.

Dem Fachmann sind viele Vektoren bekannt, die verwendet werden können, um exogene Gene in Säugetierzellen zu übertragen. Die Vektoren können episomal sein, z.B. Plasmide, Virus Vektoren wie z.B. Zytomegalovirus, Adenovirus usw. Außerdem können die Gebe durch homologe Rekombination oder durch zufällige Integration ins Zielzellgenom integriert werden, wie z.B. von Retrovieren abgeleitete Vektoren wie MMLV, HIV-1, ALV, usw.

Anhand von in vitro Assays oder Screenings können aktive Faktoren der Treg-Zellen analysiert werden. Es können auch Co-cutture Assays durchgeführt werden, um die Veränderungen von Tregs zu studieren, die die Vermehrung von nonnalen T Zellen einschließlich CD4 T als auch CD8 T zu suprimieren. Interaktionen mit dendritischen Zellen und anderen Antigen determinierten Zellen können ebenfalls untersucht werden. Die nach der Erfindung separierten regulatorischen Zellen können das Ausgangsmaterial einer großen Vielfalt von unterschiedlichen Analysen sein, z.B. von immunoassays für Proteinbindungsstudien, Bestimmungen des Zellwachstums, Differenzierung und funktionelle Aktivität, Produktion von Hormonen usw.

Die Figuren stellen folgendes dar:
Figuren 1a und 1b
   4-1BB *wird wird früh nach Aktivierung spezifisch von regulatorischen T-Zellen ausgeprägt*
   Verschiedene Subpopulationen (I-IV) peripherer Th-Zellen (A) wurden mittels FACS isoliert aus PBMC (B) und an Aliquots intrazelluläre Analysen des für regulatorische T-Zellen spezifischen Transkriptionsfaktors Foxp3 durchgeführt (C). Die isolierten Subpopulationen wurden dann mit PMA / lonomycin in vitro für unterschiedlich lange Zeitspannen (0, 4 und 8 Stunden) stimuliert und die Expression von 4-1 BB mittels FACS analysiert (D).
Figur 2
   *Der für regulatorische T-Zellen* spezifische *Transkriptionsfaktor* Foxp3 wird *nach Aktivierung* spezifische *in 4-1BB⁺ Th-Zellen ausgeprägt (RNA-Analyse)*
   PBMC wurden für 4h mit dem Superantigen SEB in vitro stimuliert und nachfolgend mit FACS 4-1BB⁺ und 4-1 BB⁻ Th-Zellen isoliert Aus den isolierten Zellen wurde RNA isoliert und mittels RT-PCR die Expression von Foxp3 analysiert.
Figur 3
   *Der für regulatorische T-Zellen spezifische Transkriptionsfaktor Foxp3 wird nach Aktivierung spezifisch in 4-1BB⁺ Th-Zellen ausgeprägt (Protein-Analyse)*
   CD4⁺ Th-Zellen wurden aus PBMC isoliert und für 4h mit dem PMA / Ionomycin in vitro stimuliert. Nachfolgend wurden mit FACS 4-1BB⁺ und 4-1BB⁻ Th-Zellen isoliert. Die isolierten Zellen wurden dann intrazellulär mit Foxp3-spezifiscen Antikörpern markiert.
Figur 4
   *Koexpression von 4-1 BB und Foxp3 früh* nach *Aktivierung*
   CD4⁺ Th-Zellen wurden aus PBMC isoliert und für 4h mit dem PMA / lonomycin in vitro stimuliert. Nachfolgend wurden die Zellen extrazellulär mit Antikörpern gegen CD69 (Aktivierungsmarker) und 4-1BB markiert. Nachfolgend wurde intrazellulär mit Foxp3-spezifischen Antikörpern markiert. Im linken Analyseplot ist das elektronische Analysefenster für CD69⁺ aktivierte Th-Zellen angezeigt. Im rechten Analyseplot ist die Koexpression von 4-1 BB und Foxp3 in CD69⁺ aktvierten Th-Zellen dargestellt
Figuren 5 bis 7:
   Spezifität und in vitro Suppression TSST-1-spezifischer CD25⁺⁺ Treg nach Isolierung über TSST-1-induzierte 4-1 BB Expression
Figur 5:
   Ruhende regulatorische CD25⁺⁺ CD4⁺ T-Zellen wurden mittels FACS aus PBMC eines gesunden Spenders isoliert und mit autologen CD4⁻ CD25⁻ PBMC in einem Verhältnis von 1:2 mit 1µg/ml TSST-1 für 4h bei 37°C kultiviert. Nachfolgend wurden mittels FACS 4-1BB⁺ und 4-1BB⁻ CD4⁺ CD69⁺ T-Zellen isoliert.
Figur 6:
   4-1BB⁺ und 4-1BB⁻ CD4⁺ CD69⁺ T-Zellen wurden nach FACS-Sortierung direkt mit Antikörpern gegen Foxp3, Vbeta2 und Vbeta17 markiert und analysiert
Figur 7:
   4-1BB⁺ CD4⁺ CD69⁺ T-Zellen wurden nach FACS-Sortierung für 6 Tage in Medium und 10%FCS kultiviert. Nachfolgend wurden sie mit CFSE-markierten naiven CD45RA⁺ CD4⁺ T-Zellen und CD4⁻ PBMC im Verhättnis 1:1:1 für 4 Tage in der Gegenwart von TSST1 0,5µg/ml kultiviert Zur Kontrolle wurden CFSEmarkierte naive CD45RA⁺ CD4⁺ T-Zellen und CD4' PBMC im Verhältnis 2:1 für 4 Tage in der Gegenwart von TSST1 0,5µg/ml kultiviert.

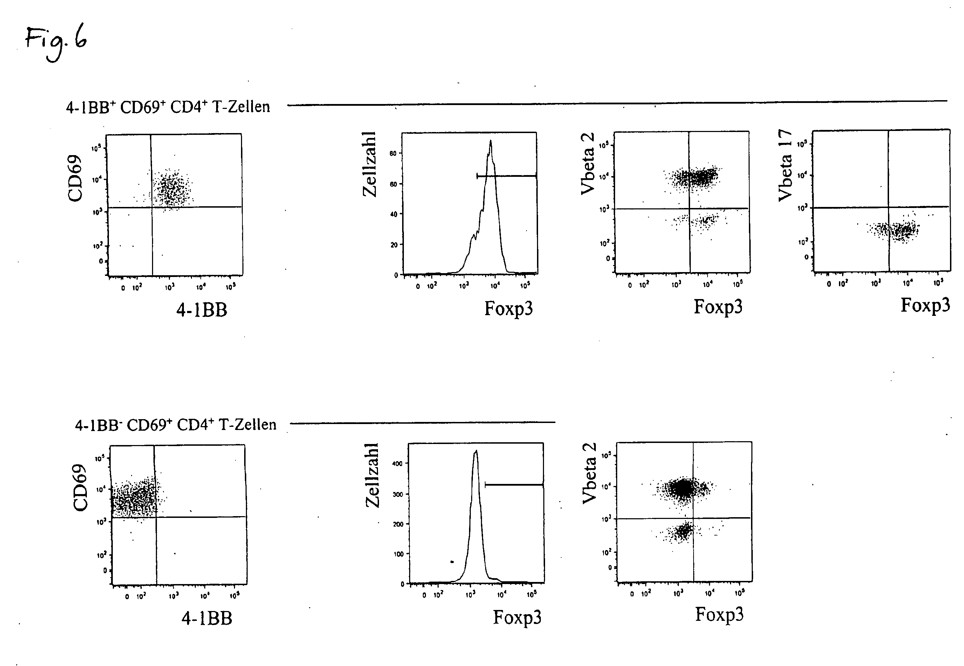

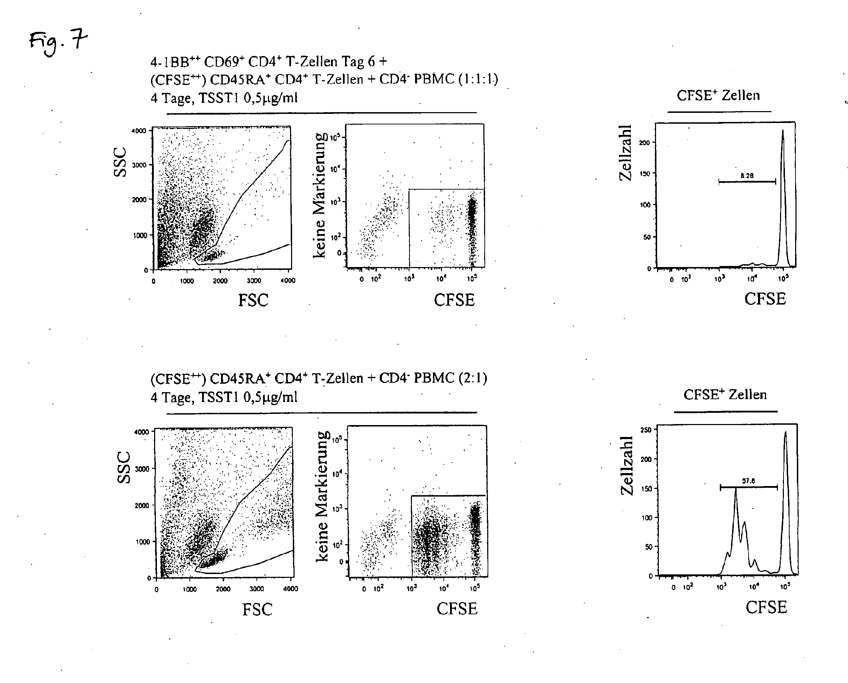

Die ergänzten Daten demonstrieren zum einen die Spezifität der durch eine Stimulation mit Antigen (hier TSST-1) induzierten 4-1BB Expression. In dem hier gewählten Zeitfenster von 4 Stunden, wird 4-1B8 fast ausschließlich von Vbeta2⁺ Foxp3⁺ Zellen ausgeprägt, während ein weiterer in Literatur beschriebener T-Zett-Aktivierungsmarker (CD69) auch von Foxp3⁻ Zellen ausgeprägt wird (Fig. 6). Zudem sind unter den isolierten 4-1BB⁺ Zellen fast keine Vbeta17⁺ Zellen detektierbar (Fig. 6). Figur 7 demonstriert dann das Suppressionspotential von TSST-1-spezifischen Treg nach Isolierung und in vitro Kultivierung. Die Proliferation von TSST-1-spezifischen naiven T-Zellen wird von TSST-1-spezifischen Tregs von über 55% auf weniger als 10% unterdrückt (Fig. 7).

## Patentansprüche

1. In vitro Verwendung von einem 4-1 BB Rezeptor als ein diskriminativer Marker zur Identifizierung und/oder Separation aktivierter regulatorischer CD4+ CD25+ Th-Zellen (Treg) von aktivierten CD4+ CD25- Th-Zellen,
in einem Zeitraum von 3 bis 8 Stunden, bevorzugt 4 Stunden, nach einer Aktivierung,
wobei der 4-1 BB Rezeptor von den aktivierten regulatorischen CD4+ CD25+ Th-Zellen (Treg) ausgeprägt wird.

2. Verwendung nach einem der Ansprüche 1 oder 2 ,
**dadurch gekennzeichnet, dass**
die zeitliche Ausprägung des Rezeptors als ein diskriminativer Marker für aktivierte regutatorische Th-Zellen durch mehrfache, bevorzugt regelmäßige Kontrollen; insbesondere in im wesentlichen 10 bis 40-minütigen Abständen, besonders bevorzugt in im wesentlichen 30-minütigen Abständen bestimmbar ist.

3. Verwendung nach Anspruch 1,
**dadurch gekennzeichnet, dass**
CD4+, CD25+ und/oder FoxP3+ regulatorische Th-Zellen identifiziert und/oder separiert werden.

4. Verwendung nach einem der vorherigen Ansprüche,
**dadurch gekennzeichnet, dass**
Antigen-spezifische regulatorische Th-Zellen identifiziert und/oder separiert werden.

5. Verwendung nach einem der vorherigen Ansprüchen,
**dadurch gekennzeichnet, dass**
die Expression des 4-1 BB Rezeptors nach und/oder während einer Stimulation gemessen wird.

6. Verwendung nach einem der vorherigen Ansprüchen,
**dadurch gekennzeichnet, dass**
die regulatorischen Th-Zellen nach und/oder während einer Stimulation der Zellen im Zellgemisch identifiziert und/oder separiert werden.

7. Verwendung nach einem der vorherigen Ansprüchen
**dadurch gekennzeichnet, dass**
die Zellen durch Wachstumsfaktoren, Antikörpern und/oder Liganden stimuliert werden.

8. Verwendung nach einem der vorherigen Ansprüchen,
**dadurch gekennzeichnet, dass**
Antigene, Liganden und/oder Antikörper zur Detektion des 4-1 BB Rezeptors verwendet werden.

9. Verwendung nach einem der vorherigen Ansprüchen,
**dadurch gekennzeichnet, dass**
die Antigene, Liganden und/oder Antikörper zur Detektion des 4-1 BB Rezeptors mit einem fluoreszierenden Stoff, Haptenen und/oder magnetischen Partikeln gekoppelt sind.

10. Verwendung nach einem der vorherigen Ansprüche,
**dadurch gekennzeichnet, dass**
der 4-1 BB Rezeptor Antikörper mit einem fluoreszierenden Stoff, Haptenen und/oder mit magnetischen Mikropartikeln gekoppelt ist.

11. Verwendung nach einem der vorherigen Ansprüche,
**dadurch gekennzeichnet, dass**
lebende regulatorische Th-Zellen identifiziert und/oder separiert werden.

12. Verwendung eines Antikörpers zur Detektion des 4-1 BB Rezeptors in der Verwendung gemäß einem der Ansprüche 1 bis 11.

13. Verwendung eines Kits,
umfassend einen Antikörper zur Detektion des 4-1 BB Rezeptors in der Verwendung gemäß einem der Ansprüche 1 bis 11.

## Claims

1. In-vitro use of a 4-1BB receptor as a discriminative marker for the identification and/or separation of activated regulatory CD4+ CD25+ Th-Cells (Treg) from activated CD4+ CD25- Th-Cells,
in a time frame from 3 to 8 hours, preferably 4 hours, after an activation, whereby the 4-1BB receptor is expressed by the activated regulatory CD4+ CD25+ Th-Cells (Treg).

2. Use according to one of claims 1 or 2,
characterized that
the temporal expression of the receptor as a discriminative marker of activated regulatory Th-Cells may be determined through multiple, preferably regular controls, in particular at essentially 10 to 40 minute intervals, particularly preferred at essentially 30 minute intervals.

3. Use according to claim 1,
**characterized in that**,
CD4+ CD25+ and/or FoxP3+ regulatory Th-Cells are identified and/or separated.

4. Use according to one of the preceding claims,
characterized that
antigen-specific regulatory Th-Cells are identified and/or separated.

5. Use according to one of the preceding claims,
characterized that
the expression of the 4-1BB receptor is measured after and/or during a stimulation.

6. Use according to one of the preceding claims,
characterized that
the regulatory Th-cells are identified and/or separated after and/or during a stimulation of cells in a cell mixture.

7. Use according to one of the preceding claims
characterized that
the cells are stimulated through growth factors, antibodies and/or ligands.

8. Use according to one of the preceding claims
characterized that
antigens, ligands and/or antibodies are used for the detection of the 4-1BB receptor.

9. Use according to one of the preceding claims
characterized that
the antigens, ligands and/or antibodies are coupled with a fluorescent substance, haptenes and/or magnetic particles for the detection of the 4-1BB receptor.

10. Use according to one of the preceding claims
characterized that
the 4-1BB receptor antibody is coupled with a fluorescent substance, haptenes and/or with magnetic microparticles.

11. Use according to one of the preceding claims
characterized that
living regulatory Th-cells are identified and/or separated.

12. Use of an antibody for the detection of the 4-1BB receptor in a use according to one of claims 1 to 11.

13. Use of a kit,
comprising an antibody for the detection of the 4-1BB receptor in the use according to one of claims 1 to 11

## Revendications

1. Utilisation in vitro d'un récepteur 4-1 BB comme marqueur de discrimination en vue de l'identification et/ou de la séparation de lymphocytes auxiliaires Th CD4+ CD25+ régulateurs (Treg) activés de lymphocytes auxiliaires Th CD4+ CD25- activés en un laps de temps de 3 à 8 heures, de préférence de 4 heures, après une activation, le récepteur 4-1 BB étant exprimé par les lymphocytes auxiliaires Th CD4+ CD25+ régulateurs (Treg) activés.

2. Utilisation selon l'une quelconque des revendications 1 ou 2, **caractérisée en ce que** l'expression dans le temps du récepteur en tant que marqueur de discrimination pour des lymphocytes auxiliaires Th régulateurs activés peut être déterminée par des contrôles multiples, de préférence réguliers, en particulier à des intervalles essentiellement de 10 à 40 minutes, de manière particulièrement préférée à des intervalles essentiellement de 30 minutes.

3. Utilisation selon la revendication 1, **caractérisée en ce que** des lymphocytes auxiliaires Th CD4+, CD25+ et/ou FoxP3+ régulateurs sont identifiés et/ou séparés.

4. Utilisation selon l'une quelconque des revendications précédentes, **caractérisée en ce que** des lymphocytes auxiliaires Th régulateurs spécifiques d'antigènes sont identifiés et/ou séparés.

5. Utilisation selon l'une quelconque des revendications précédentes, **caractérisée en ce que** l'expression du récepteur 4-1 BB est mesurée après et/ou pendant une stimulation.

6. Utilisation selon l'une quelconque des revendications précédentes, **caractérisée en ce que** des lymphocytes auxiliaires Th régulateurs sont identifiés et/ou séparés après et/ou pendant une stimulation des lymphocytes dans le mélange de lymphocytes.

7. Utilisation selon l'une quelconque des revendications précédentes, **caractérisée en ce que** les lymphocytes sont stimulés par des facteurs de croissance, des anticorps et/ou des ligands.

8. Utilisation selon l'une quelconque des revendications précédentes, **caractérisée en ce qu'**on utilise des antigènes, des ligands et/ou des anticorps pour la détection du récepteur 4-1 BB.

9. Utilisation selon l'une quelconque des revendications précédentes, **caractérisée en ce que** les antigènes, les ligands et/ou les anticorps pour la détection du récepteur 4-1 BB sont couplés à une substance fluorescente, des haptènes et/ou des particules magnétiques.

10. Utilisation selon l'une quelconque des revendications précédentes, **caractérisée en ce que** l'anticorps anti-récepteur 4-1 BB est couplé à une substance fluorescente, des haptènes et/ou des particules magnétiques.

11. Utilisation selon l'une quelconque des revendications précédentes, **caractérisée en ce que** des lymphocytes auxiliaires Th régulateurs vivants sont identifiés et/ou séparés.

12. Utilisation d'un anticorps pour la détection du récepteur 4-1 BB dans l'utilisation selon l'une quelconque des revendications 1 à 11.

13. Utilisation d'un kit, comprenant un anticorps pour la détection du récepteur 4-1 BB dans l'utilisation selon l'une quelconque des revendications 1 à 11.
